# EUROPEAN PATENT SPECIFICATION

(11) **EP 1 205 163 B1**
(45) Date of publication and mention of the grant of the patent: **15.04.2009**
(21) Application number: 01204267.7
(22) Date of filing: 07.11.2001
(51) Int. Cl.: A61F 2/36, A61B 17/17, A61B 17/16

(54) **Fastening element for an implant, in particular a hip prosthesis**
Befestigungselement für ein Implantat, insbesondere eine Hüftprothese
Elément de fixation pour un implant, en particulier une prothèse de hanche

(30) Priority: 07.11.2000 NL 1016551; 07.11.2000 US 246557 P
(43) Date of publication of application: 15.05.2002
(73) Proprietor: Hoffman, Erik Leonard, 4702 HL Roosendaal (NL)
(72) Inventor: Hoffman, Erik Leonard, 4702 HL Roosendaal (NL)
(74) Representative: Verhees, Godefridus Josephus Maria

(56) References cited:
- EP-A- 0 094 829
- EP-A- 0 099 167
- WO-A-00/09038
- WO-A-86/03962
- WO-A-89/11837
- WO-A-93/16663
- WO-A-98/07393
- DE-A- 3 538 346
- DE-A- 3 607 824
- DE-A- 3 917 285
- DE-A- 19 725 269
- DE-A- 19 834 277
- DE-A- 19 904 126
- DE-B- 1 164 019
- FR-A- 2 353 275
- FR-A- 2 478 462
- FR-A- 2 674 122
- GB-A- 2 033 755
- NL-A- 9 202 201
- US-A- 5 376 126
- US-A- 5 702 474

## Description

This invention relates to a fastening element for an implant.

This invention relates in particular to a fastening element for a hip prosthesis. Such a fastening element is known from EP-A-0 099 167.

This known fastening element has a supporting element and a pin whereby the angle between the longitudinal axis of the pin and the main surface of the supporting element is generally 90 degrees.

This known fastening element has as a disadvantage that it requires removal of relatively much bone material. In practice, this means that if after a number of years such a fastening element exhibits play, replacement of the fastening element has become virtually impossible, since so much bone material has been removed. A further disadvantage of such a fastening element is that forces and moments applied thereto are unfavourably transmitted, so that bone tissue disappears as a result of the well-known stress shielding phenomenon.

The object of the invention is to provide a fastening element of the type described in the preamble, which avoids the disadvantages mentioned while maintaining the advantages thereof. To that end, a fastening element according to the invention is characterized by the features according to claim 1.

For the use of the fastening element according to the invention less bone material has to be sawn off than with the known prosthesis. With a fastening element according to the invention, in a particularly advantageous manner, a fastening for a hip prosthesis can be obtained with a normal angle between the longitudinal axis of the femur and the longitudinal axis of the neck, the so-called CCD angle, even for a coxa vara and coxa valga.

The pin can be driven into a bone, such that the supporting element comes to lie against a sawn-off face of the bone, while the longitudinal axis of the pin includes an angle therewith. The main surface of the supporting element is then to be understood to include a surface approximately parallel to the sawing plane along which a bone portion has been sawn off. With such a fastening element, the advantage is achieved that a particularly advantageous force transmission can be obtained, while the pin can be driven into a bone in a suitable manner, such that it is surrounded on all sides by sufficient spongy and/or cortical bone.

To clarify the invention, exemplary embodiments thereof will be further elucidated with reference to the drawings. In the drawings:
Figs. 1A-1C show in three steps how a fastening element for a prosthesis according to the invention is fitted;
Fig. 2 shows in top plan view a femur according to Fig. 1B;
Fig. 3 shows the head of a femur with a fastening device according to the invention, in sectional view along the line III-III in Fig. 2;
Figs. 4A-4C shows three cross sections of a pin of a fastening device according to the invention;
Fig. 5 shows in top plan view a tool for use for placing a fastening device according to the invention;
Figs. 5A and 5B show the tool according to Fig. 5 in sectional view along the lines VA-VA and VB-VB, respectively;
Fig. 5C shows a sawing tool for use in the invention, in mounted condition;
Fig. 6 shows on an enlarged scale a portion of a fastening device according to the invention with fixing means;
Fig. 7 shows in a view similar to Fig. 3 an alternative embodiment of an assembly according to the invention;
Fig. 7A shows in sectional side elevation an alternative embodiment of a fastening element according to the invention;
Fig. 8 shows a side elevation of a further alternative embodiment of a fastening element according to the invention;
Fig. 9 shows a still further alternative embodiment of a fastening element according to the invention, placed on an abutment surface; and
Fig. 10 schematically shows a block chisel for use in the invention.

In this description, the same or corresponding parts have the same or corresponding reference numerals. In the exemplary embodiments shown, a hip prosthesis, in any case a fastening device therefor, is shown, with an upper end of a femur. However, in the same or a comparable manner, other prostheses or ortheses can be fixed, for instance a knee prosthesis, shoulder prosthesis or the like.

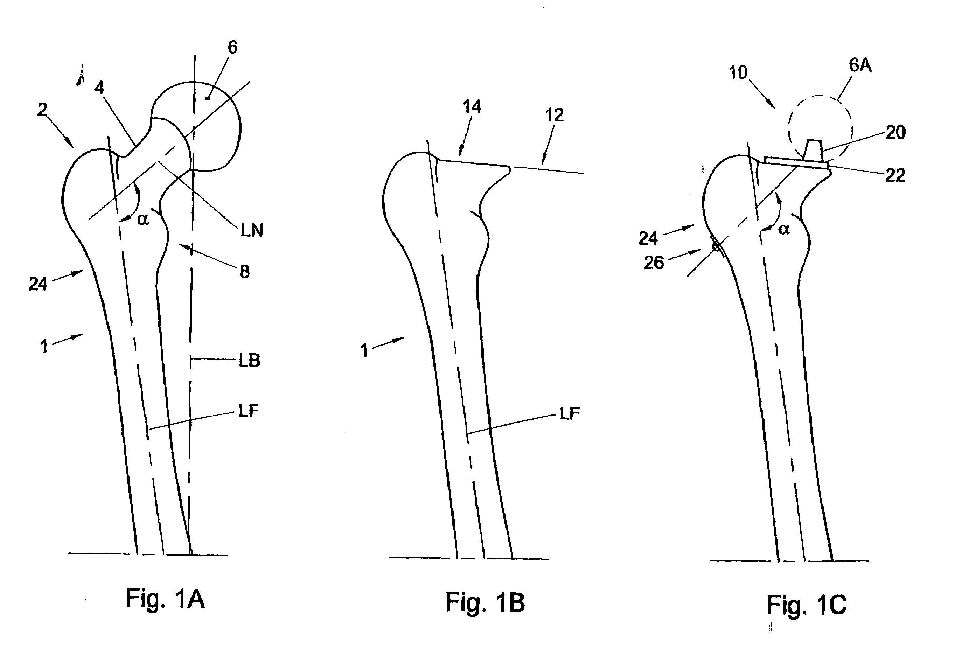

Fig. 1a shows approximately in front view the upper part of a femur 1. At the top, the femur is provided laterally with a great trochanter 2 and on the proximal side with a head 6 carried by a neck 4. The neck has a longitudinal axis LN which includes an angle α with the longitudinal axis LF of the femur 1. The projection of this angle on the mediolateral (ML) plane, angle α, is usually designated as the CCD angle and is normally between about 125° and 145°. If the CCD angle is less than 125°, a coxa vara is involved; if the angle is greater than 145 °, a coxa valga is involved. The angle α will hereinafter be designated as the CCD angle, while in each case the actual angles, for instance between pin and supporting element, can be derived from this. Represented under the neck 4 is the lesser trochanter 8.

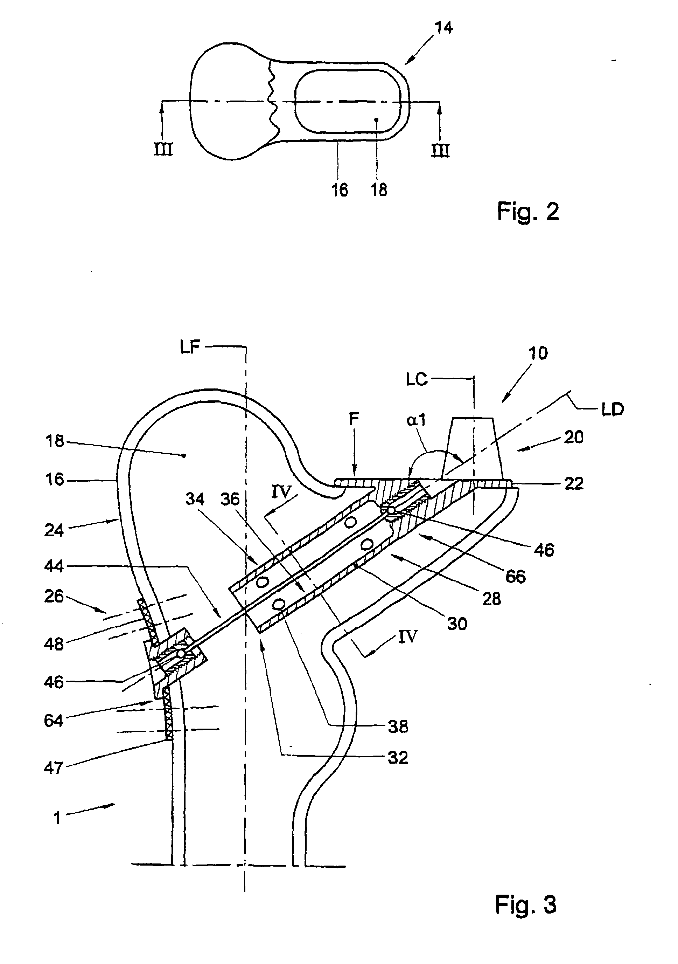

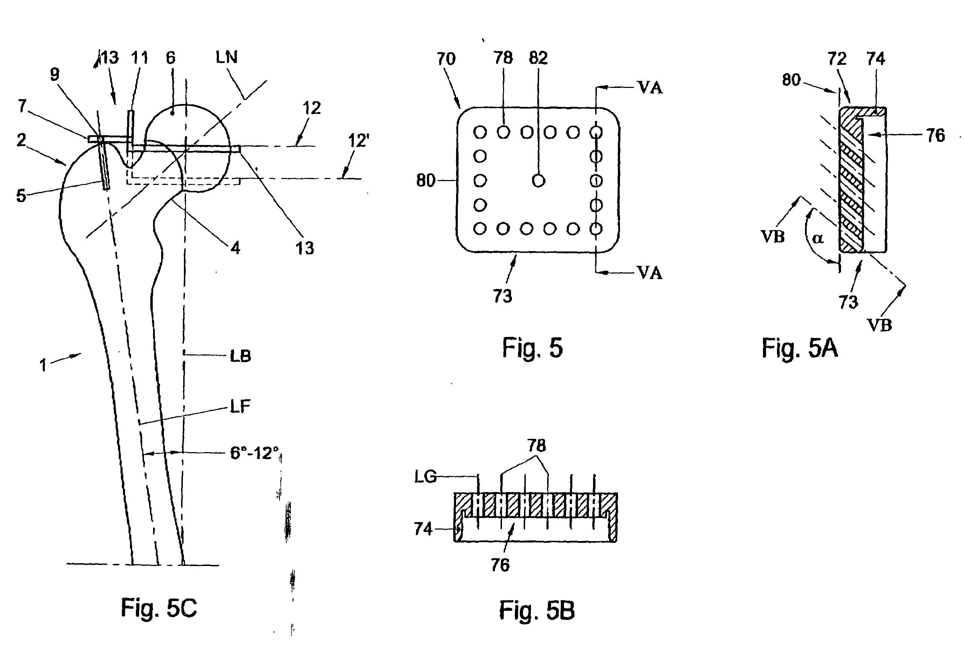

For placement of a fastening element 10 according to the invention, as schematically represented in Fig. 1C, the head 6 with at least a portion of the neck 4 needs to be sawn off. To that end, as shown in Fig. 1B, the neck 4 is sawn off along a sawing plane 12, thereby forming an abutment surface 14. A tool to be used for that purpose will be described hereinafter in more detail with reference to Fig. 5C. It is then preferred, at least in a hip prosthesis as shown in Fig. 1, to choose the sawing plane 12 such that it extends approximately at right angles to the load axis LB. The load axis LB is a straight line drawn through the center of the hip head and the intercondylar space of the knee. This load axis LB usually includes an angle between 6° and 12° with the longitudinal axis of the femur. With a femur in the normal, upright position, the abutment surface 14 will therefore include an angle between approximately 6° and 12° with the horizontal, as shown in approximation in 1B. Fig. 2 shows a top plan view of the femur 1 with abutment surface 14 according to Fig. 1B. Fig. 2 clearly shows that the abutment surface 14 comprises a supporting edge 16 of cortical bone, which encloses a central surface of spongy bone 18.

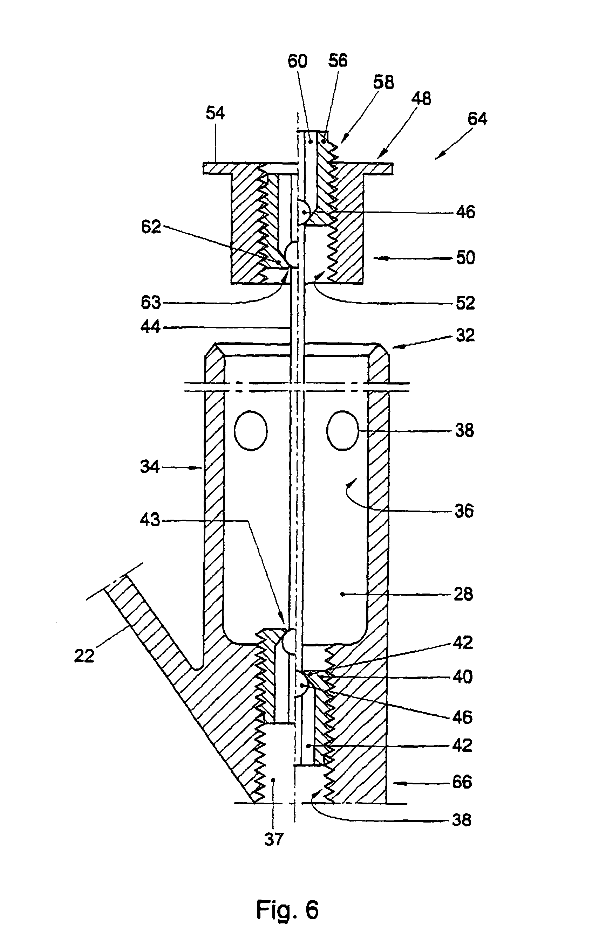

Fig. 1C shows a fastening element 10, fitted in a femur 1. Of the fastening element 10, a coupling element 20 is visible, mounted on a plate-shaped supporting element 22 which abuts on the cortical supporting edge 16 and the spongy bone 18. Fig. 3 shows, in a cross section along the line III-III in Fig. 2, the upper part of femur 1, with a fastening element 10 as shown in Fig. 1C. Fig. 1C shows on the lateral side, against the lateral cortex 24, a portion of the fixing means 26 by means of which the fastening element 10 is pulled against the abutment surface 14, at least the supporting edge 16. This will be described in more detail hereinafter. A pin 28, which extends from the supporting element 22, has been driven into the spongy bone 18, so that a particularly good fastening of the fastening element 10 has been obtained. The pin 28 is substantially hollow and has a circumferential wall 30 of relatively thin material. As appears clearly from Fig. 6, the lower longitudinal edge 32 of the circumferential wall 30 may be of sharp design, so that it can cut into the spongy bone. This will be further discussed hereinafter.

Referring in particular to Fig. 3, there is shown a cross section of a fastening element 10 comprising a plate-shaped supporting element 22 which is substantially flat and extends on at least two opposite sides and preferably around an upper end of a pin 28. The shape of the supporting element 22 in top plan view has been chosen such that it can fittingly abut against the supporting edge 16 without undesirably extending beyond it unduly. As a consequence, the supporting element 22 can fittingly abut against the abutment surface 14, so that vertical forces, in any case forces F approximately at right angles to the plane of the supporting element 22, can be properly transmitted to the cortical bone and to some extent to the spongy bone. The pin 28 in the embodiment shown is of substantially hollow design and has a longitudinal axis LP which includes an angle α₁ with the plane of the supporting element 22, which angle approximately corresponds to the CCD angle as shown in Fig. 1A. When placing the fastening element 10, the pin is driven in axial direction along the longitudinal axis LP into the spongy bone 18, with the driving angle being chosen such that it includes the above-mentioned angle α with the longitudinal axis LF of the femur 1, so that the supporting element 22 will come to lie flat against the abutment surface 14. It will be clear that the angle α₁ can be suitably chosen in each case, depending on the CCD angle of the femur in question and the angle of the abutment surface 14 with respect to the longitudinal axis LF of the femur. In the embodiment shown, the coupling element 20 is designed in a known manner as a cone tapering in the direction away from the supporting element 22. The cone 20 has a longitudinal axis LC which likewise includes approximately the CCD angle with the longitudinal axis LP of the pin 28. The longitudinal axis LC of the cone 20 is then located approximately parallel to the longitudinal axis LF of the femur 1. The distance between the longitudinal axis LF of the femur 1 and the longitudinal axis LC of the cone 20 then substantially determines the offset. To be able to vary this offset, the coupling element 20 can be arranged to be displaceable with respect to the supporting element 22, but also a number of different supporting elements 22, at any rate fastening elements 10, may be provided, from which a suitable embodiment can be chosen in each case.

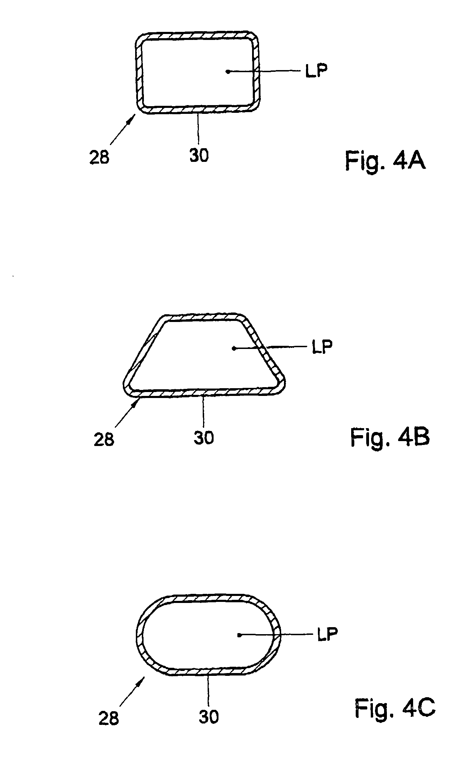

Figs. 4A-C show three cross sections of the pin 28 along the line IV-IV in Fig. 3, by way of example. Each of these cross sections is other than rotation-symmetrical with respect to the longitudinal axis LP, so that the pin 28 can take up torsional forces in the plane of the drawing, while being restrained from rotation about the longitudinal axis LP. Depending on the shape of the abutment surface 14 and the implant in question, in each case a suitable cross section can be chosen, which choice will be immediately clear to one skilled in the art. In particular current cross sections of implants can then be employed. In the hollow design of the pin, it has an at least substantially constant cross section.

When the pin, designed as a hollow pin 28 in Fig. 3, is driven into the spongy bone 18, spongy bone 18 will be present both on the outside 34 and on the inside 36 of the pin 28. It is then preferred that the outside 34 and/or the inside 36 are so designed that the spongy bone can grow into it, at least can bond to it. Optionally, openings 38 or slots (not shown) or the like are provided, into which or through which the spongy bone 18 can grow. This yields a proper retention of the pin 28 and hence of the fastening element 10. In Fig. 3, further, fixing means 26 are shown, by means of which a still better fixation of the fastening element 10 can be obtained. In Fig. 6, on a slightly enlarged scale, a portion of a fastening element 10 with the fixing means 26 is shown in more detail.

On the side proximal to the supporting element 22, the pin 28 is provided with a bore 37 with internal screw thread 38. In the bore 37, a first bush 40 has been screwed, provided with an inner hexagon 42. The first bush 40 has a bottom 42 with a central opening through which a wire element 44 extends. This wire element 44 is provided at both ends with a wire head 46 which cannot pass the opening 43 in the bottom 42. This wire head 46 is, for instance, upset, welded or soldered on, but may also be formed by bending over the wire material. By screwing the first bush 40 in the bore 37 inwards or outwards, the wire head 46 can be displaced axially. At the opposite end, a fastening pin 48 is provided, which comprises a cylindrical part 50 with internal screw thread 52 and is provided, on a side facing outwards during use, with a circumferential flange 54 extending outwards. In the internal screw thread 52, a second bush 56 with external screw thread 58 and an inner hexagon 60 is provided, comparable to the first bush 40, which may or may not be provided with a bottom 62 with a central opening 63 through which the wire element 44 extends and through which the wire head 46 cannot pass. Again, the wire head 46 can be displaced axially by screwing the second bush 56 into or out of the supporting element 48. The supporting element 48 and the second bush 56 jointly form first tensioning means 64; the bore 37 with internal screw thread 38 and the first bush 40 form second tensioning means 66.

To mount the fastening element 10, a hole is drilled through the cortical bone 16, parallel to and concentrically with the longitudinal axis LP of the pin 28. Through this hole, the first tensioning means 64 with the wire element 44 are inserted, with the second bush 56 being screwed in to a maximum. The wire element 44 is then preferably stretched and manufactured from memory material. On the side remote from the first tensioning means 64 is the wire head 46 with the first bush, which bush is screwed into the fastening element 10, whereafter the two bushes 40, 56 are screwed apart and the wire element 44 is tensioned between them. The memory material for the wire element 44 in this embodiment is preferably chosen such that at body temperature, for instance approximately 37°, it exhibits the tendency to resume its original, undeformed position. Prior to placement, the wire element has been elongated, for instance, by about 8%. As a result, accordingly, under the influence of the body temperature, a tension will arise in the wire element, so that a still better fastening is obtained.

In an alternative embodiment, the wire element is manufactured from memory metal having a considerably higher switch temperature, for instance 75° to 80°. In this embodiment, the wire element, after it has been fitted in the femur, is engaged with gripper tongs from the lateral cortex and stretched, for instance by about 8% of its initial length, whereafter the two bushes 40, 56 are screwed apart. If the wire element is subsequently released, it will exert a tensile stress on the two bushes, which tensile stress is relatively constant, also when the bushes will be pulled towards each other to some extent. The wire element will behave superelastically.

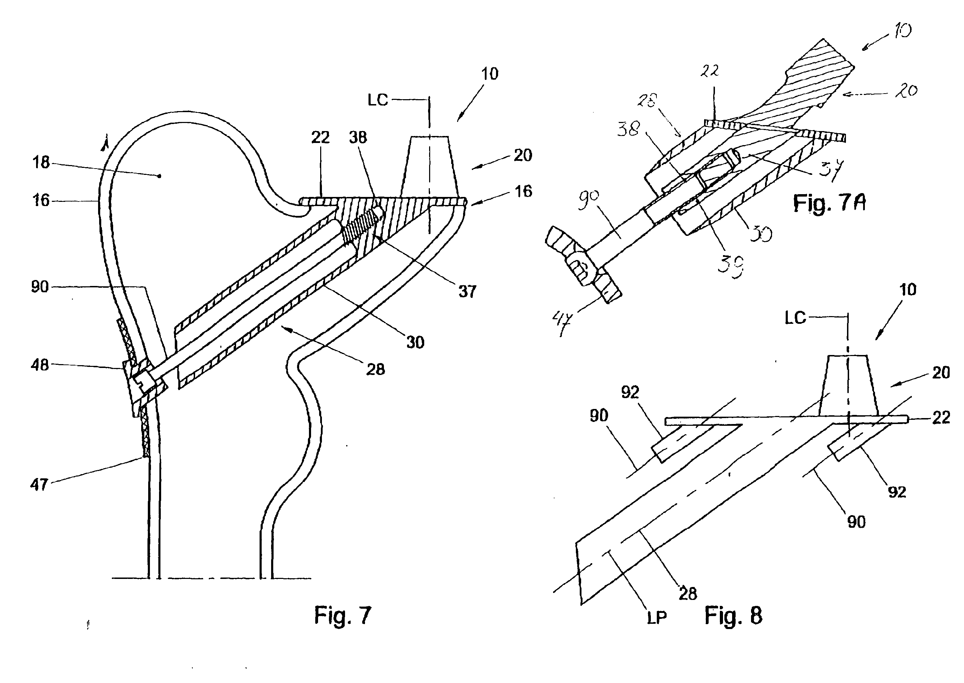

Fig. 7 shows an alternative embodiment for fastening the fastening element 10, in which a screw 90 inserted from the lateral cortical side into the femur, through a bush 48, which bolt 90 has been screwed into the opening 37 provided with screw thread 38 and located under the supporting element 22. Subsequently, the screw 90 has been tightened such that the desired bias is obtained. Between the bush 48 and the cortical bone 16 a supporting plate 47 is arranged, so that the occurring stresses and forces are distributed over a relatively large surface. A comparable plate 47 is shown in Fig. 3.

The opening 37 can also be designed as a shaft 39, extending from the supporting element 22 into the hollow pin 28, as shown in Fig. 7A, while the inside of the shaft is provided with screw thread 38. By virtue of such a shaft 39, the screw 90 can be screwed down in the fastening element 10 over a greater length, and a greater setting range is obtained for setting a suitable bias in the screw 90. Moreover, the shaft 39 enlarges the bonding surface for spongy bone situated between this shaft and the pin 28. As a result, a still better stable fixation of the fastening element 10 in the femur can be accomplished.

Various parts, such as the screw 90, the supporting plate 47 and/or the shaft 39 can be manufactured from a resorbable material. This is especially advantageous in the embodiments of Figs. 7 and 7A, but can also be used in the other embodiments. Furthermore, the or each part of the fastening element 10 which, in mounted condition, is in contact with bone material is preferably provided with a coating which enables, preferably stimulates, bone ingrowth, for instance an RGD coating. Such a coating is applicable in all embodiments shown.

In Fig. 8 an alternative embodiment of a fastening element 10 according to the invention is shown, in which the pin 28 can be of both hollow and massive design. At some distance from the pin, there are provided against the underside of the supporting element 22 a number of bushes 92, for instance four, placed adjacent the corners of the supporting element 22. The bushes 92 are provided with internal screw thread and have a centerline which extends preferably approximately parallel to the centerline LP of the pin 28. In the bushes 92, bolts 90 can be screwed, in a manner comparable to that shown and described with reference to Fig. 7. Such a positioning of the bolts 90 provides a still greater stability for the supporting element 22.

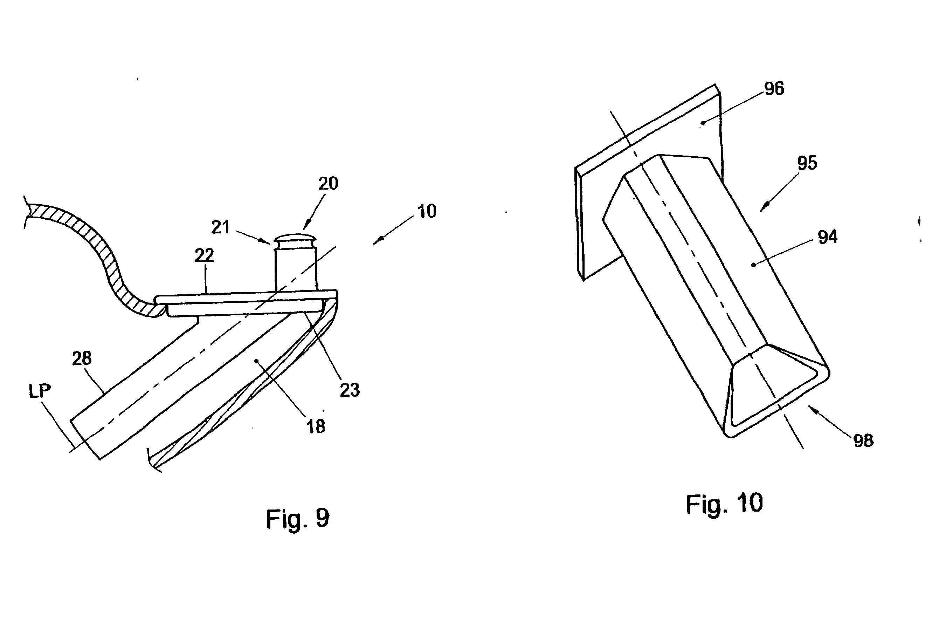

In Fig. 9 a further alternative embodiment is shown, in which under the supporting element 22 a thickening 23 is arranged, which is located to some extent within the contours of the supporting element 22. When placing the supporting element, the thickening 23 is driven slightly into the spongy bone 18, which is thereby densified to some extent, while the part of the supporting element 22 extending outside the thickening abuts against the cortical edge 16. As a result, a still better locking is obtained. In this embodiment, the coupling means 20 is designed as a cylindrical pin, provided with a snap edge 21, on which, for instance, a ball 6A can be fixed, optionally provided with clamping means cooperating with the snap edge 21.

In an advantageous embodiment, the longitudinal axis LP of the pin 28 extends through the heart of the head 6A placed on the coupling element 20. Occurring forces and moments are thereby further reduced.

In Fig. 5C, a sawing tool 3 is shown, suitable for use in sawing off at least a part of the head 6 and the neck 4. This sawing tool 3 comprises a pin 5 which can be driven into the great trochanter, along the longitudinal axis LF of the femur 1. To that end, first an appropriate hole is drilled. On the pin 5 a bridge piece 7 is carried, coupled thereto by means of a pivotal coupling 9, so that the position of the bridge piece 7 can be set with respect to the longitudinal axis LF and the load axis LB of the femur 1. A sawing table 13 is carried by the bridge piece 7 with the aid of an arm, such that the sawing table 13 is carried on one side or both sides of the head 6. To that end, the table 13 can be made of, for instance, fork-shaped design. As is indicated in Fig. 5C, the height of the plane 12, 12' of the sawing table 13 can be set, so that the head 6 and neck 4 can be sawn off at the proper height, with the saw being guided by the sawing table 13. The sawing table 13 is set such that the plane 12, 12' extends approximately at right angles to the load axis LB. After the head 6 and the neck 4 have been correctly sawn off at the proper height, the sawing tool 3 is removed by pulling the pin 5 out of the femur 1.

In Fig. 5, in top plan view, a tool 70 is shown, suitable for use in placing a fastening element 10 according to the invention. This tool 70 comprises a plate part 72, provided at the underside with a skirt 74 which preferably extends along three sides of the plate part 72. On the fourth side 73, the skirt 74 is interrupted, and with this side the tool 70 can be placed in the direction of the great trochanter. Preferably, the skirt 74 then lies approximately against the outside of the cortical bone 16, at least the supporting edge, while the underside 76 of the plate part 72 lies against the abutment surface 14. Thus, the tool 70 is properly positioned. In the plate part 72, a series of holes 78 are provided which in top plan view (Fig. 5) are arranged in a pattern that corresponds to the cross section of the pin 28, for instance as shown in Fig. 4A. The holes 78 each have a longitudinal axis LG, which longitudinal axes extend parallel to each other. In side elevation, as shown in Fig. 5, the longitudinal axes LG include an angle α₂ with the top surface 80 of the plate part 72, which angle α₂ approximately corresponds to the CCD angle of the femur 1 in which the tool 70 is to be used. In an elevation as shown in Fig. 5B, the longitudinal axis LG of each opening 78 extends at right angles to the surface 80. A tool 70 according to Fig. 5 can be used as follows.

After the abutment surface 14 has been formed by sawing off the neck 4, the tool 70 is laid with the plate part 72 on the abutment surface 14, with the sides 73 facing the great trochanter. Then, through the holes 78 a drill (not shown) is introduced, with which a bore is provided in the spongy bone. The longitudinal axes of the bores in the spongy bone will then be parallel to each other and to the longitudinal axes LG of the holes 78, so that in the spongy bone a pattern of bores is obtained, corresponding to the hole pattern in the tool 70. The bores then have, for instance, a depth corresponding to the length of the pin 28. Thereafter the tool 70 is removed and the pin 28, with the edge 32 leading, is driven into the spongy bone, with the edge 32 being guided by the bores since the bores and the intermediate material will constitute the path of least resistance. Thus, in a simple manner, a particularly good positioning of the pin 28 and hence of the fastening element 10 is obtained. Optionally, centrally in the plate part 72, a further opening 82 may be provided, with which a central bore for the wire element 44 can be guided. Such a central bore can then extend through the lateral cortex 24, so that it becomes visible from the outside where the supporting means 48 are to be fitted.

In Fig. 10 a block chisel 95 is shown, having a cross section which corresponds to the cross section of the pin of a fastening element 10 to be placed. The block chisel has a wall 94 which at the free longitudinal edge thereof is provided with a sharp, cutting face 98, while on the opposite side a plate 96 is provided by which the block chisel can be held and on which force can be exerted to drive the block chisel with the cutting edge 98 into the bores in the spongy bone. During use, the block chisel 95 will be driven into the bores, or directly into the spongy bone without first providing bores, to the desired depth, whereafter the block chisel 95 is removed. Into the opening thus formed, the pin is then driven.

As appears clearly from the figures and the description, fitting the fastening element 10 according to the invention is invasive to a comparatively lesser extent with respect to the method known from the prior art, since after sawing off the neck 4 no large bores need to be provided, while moreover only a limited part of the neck 4 needs to be removed. This means that relatively much bone material (both cortical and spongy) will remain undamaged. This means that when in the course of years undesired play arises in the fastening element 10, which cannot be corrected by the fixing means anymore, the fastening element can be removed and replaced with a new fastening element. Such a new fastening element can be designed, for instance, as outlined according to the invention, using, for instance, a pin 28 having a different diameter or greater length, while, if necessary, a further-reaching intervention can be applied, whereby, for instance, a fastening element as described in the preamble can be used. This means that a successive set of prostheses can be used, so that suitable prostheses can be provided for a longer time.

In the description, for the most part, a substantially hollow pin 28 has been assumed. It is also possible, however, in some cases, to design this pin to be at least substantially solid, as shown in Fig. 8, while maintaining the angle α between the supporting element 22 and the longitudinal axis LP of the pin 28 as described. In that case, too, a better force transmission of pressure forces in particular will be obtained. A substantially hollow pin, however, is preferred in most cases, since less bone needs to be removed to place it.

The invention is not limited in any way to the exemplary embodiments represented in the description and in the drawings. Many variations thereof are possible within the scope of the invention outlined by the claims.

Thus, the pin 28 may be, for instance, of double-walled design, so that the engagement surface for the spongy bone is enlarged still further. Also, different kinds of fixing means can be used, for instance a screw which is screwed into internal or external thread in the free end of the pin 28. In the exemplary embodiments shown, the pin has an end face extending approximately at right angles to the longitudinal axis LP. However, this end face may also include an angle with the longitudinal axis LP, so that driving in the pin is simplified still further. A fastening element 10 and the fixing means to be used therefor are manufactured from conventional materials for ortheses, prostheses and implants. In the exemplary embodiments shown, the pin 28 is fixedly connected with the supporting element 22 and manufactured integrally therewith. However, the pin 28 may also be manufactured separately from the supporting element and be subsequently coupled to it, for instance by welding or soldering or by means of, for instance, a thread connection, so that different kinds of pins can be connected with a supporting element, for instance at different angles. The wire element 44 is preferably manufactured from memory material, such as Nitinol, but may, of course, be manufactured from other materials.

These and many comparable variations are understood to fall within the scope of the invention as outlined by the claims.

## Claims

1. A fastening element for an implant, in particular a hip prosthesis, comprising a supporting element (22) and a pin (28) extending from the supporting element, while fixing means are provided for fixing the fastening element in a position in which the pin (28) extends at least largely into a bone (1), which supporting element (22) is substantially plate-shaped and extends on at least two sides beyond an outer longitudinal edge of the pin (28), such that during use the supporting element (22) abuts against a sawn-off surface (14) of a bone (1), and the supporting element (22) has a main surface and the pin (28) has a longitudinal axis (LP) which includes an angle (α₁) with the main surface of the supporting element (22), **characterized in that** the angle (α₁) is between 125 degrees and 145 degrees.

## Patentansprüche

1. Befestigungselement für ein Implantat, insbesondere eine Hüftprothese, das ein Stützelement (22) und einen aus dem Stützelement hervorstehenden Stift (28) umfasst, wobei Fixierungsmittel für die Fixierung des Befestigungselements in einer Position vorgesehen sind, in der der Stift (28) zumindest großenteils in einen Knochen (1) hineinreicht, und wobei das Stützelement (22) im Wesentlichen plattenförmig ausgebildet ist und auf mindestens zwei Seiten bis über einen äußeren Längsrand des Stifts (28) in der Weise hinausreicht, dass das Stützelement (22) während des Gebrauchs auf einer abgesägten Fläche (14) eines Knochens (1) aufliegt, und das Stützelement (22) eine Hauptfläche besitzt und der Stift eine Längsachse (LP) besitzt, die in einem Winkel (α₁) zu der Hauptfläche des Stützelements (22) angeordnet ist, **dadurch gekennzeichnet, dass** der Winkel (α₁) einen Wert zwischen 125 Grad und 145 Grad aufweist.

## Revendications

1. Élément d'attache pour un implant, en particulier pour une prothèse de la hanche, comprenant un élément de soutien (22) et une broche (28) qui se prolonge depuis l'élément de soutien, où des moyens de fixation sont prévus pour la fixation de l'élément d'attache dans une position où la broche (28) se prolonge au moins en majeure partie dans un os (1), lequel élément de soutien (22) a en gros la forme d'une plaque et se prolonge au moins de deux côtés jusqu'au delà d'un bord longitudinal extérieur de la broche (28), de telle manière que pendant l'utilisation l'élément de soutien (22) soit adjacent à une surface (14) sciée d'un os (1), et où l'élément de soutien (22) a une surface principale et la broche a un axe longitudinal (LP) qui forme un angle (α₁) avec la surface principale de l'élément de soutien (22), **caractérisé en ce que** l'angle (α₁) est compris entre 125 et 145 degrés.
